# EUROPEAN PATENT SPECIFICATION

(11) **EP 3 433 606 B1**
(45) Date of publication and mention of the grant of the patent: **17.06.2020**
(21) Application number: 17721844.3
(22) Date of filing: 24.03.2017
(51) Int. Cl.: G01N 31/22, C12Q 1/04, C12Q 1/18, G01N 33/04, G01N 33/493, G01N 33/52

(54) **DEVICE AND LIQUID COMPOSITION FOR THE DIAGNOSIS OF URINARY TRACT INFECTIONS**
VORRICHTUNG UND FLÜSSIGE ZUSAMMENSETZUNG FÜR DIE DIAGNOSE VON INFEKTIONEN DES HARNTRAKTS
DISPOSITIF ET COMPOSITION LIQUIDE POUR LA DIAGNOSE D'INFECTIONS DE LA VOIE URINAIRE

(30) Priority: 25.03.2016 IT UA20162034
(43) Date of publication of application: 30.01.2019
(73) Proprietor: MBS Diagnostics Ltd, London N1 7GU (GB)
(72) Inventor: ANTONINI, Giovanni, 00131 Roma (IT); MARI, Alberto, 00131 Roma (IT)
(74) Representative: Bosman, Cesare
(86) International application number: PCT/IB2017/051720
(87) International publication number: WO 2017/163224

(56) References cited:
- EP-A1- 0 611 001
- WO-A1-2013/171158
- CA-A- 681 202
- GB-A- 1 554 193

## Description

### TECHNICAL FIELD

The present invention relates to a device and to the relative reagent composition for the diagnosis of urinary tract infections.

### BACKGROUND ART

Urinary tract infections are currently one of the most common forms of bacterial infections and account for an important item of expenditure in national health care spending.

Urinary tract infections are generally diagnosed by means of a urine culture test associated with an antibiogram carried out in suitable health care facilities. The current testing method involves response times of approximately 48 hours as well as the need for specialized staff and facilities.

Alternatively, a quick testing of urinary tract infections can be carried out by means of urine test strips (also known as dipstick test), which, after having been immersed in a urine sample, change colour, thus providing the relative result. In particular, urine test strips comprise a series of reagents, which are able to react with the compounds present in urine producing a characteristic colour that can be correlated with the possible presence of a urinary tract infection. Even though this technique has the advantage of being extremely simple and of being able to provide results very quickly (approximately in 120 seconds), it cannot solve the problem alone and it needs to be combined with other clinical tests. Furthermore, comparisons with the urine culture technique have shown that the dipstick test often leads to false negative and positive results.

Other methods for the quick diagnosis of infections based on serum biomarkers, such as for example C-reactive protein, procalcitonin and others, despite being able to provide results within a few minutes, do not ensure the reliability needed if they are used for the diagnosis of urinary tract infections.

Finally, it should be pointed out that some International Authorities, such as the World Health Organization and the Centers for Disease Control and Prevention of Atlanta (Georgia, USA) establish that, for a reliable diagnosis of urinary tract infections, tests must detect the presence of quantity of bacteria greater than 100,000 per ml of urine. Therefore, the need is felt to have a reliable technique that is able to provide, more quickly than urine culture tests, diagnostic results of urine tract infections and, at the same time, is also able to highlight the antibiotic effective against the bacterium detected and, above all, without the need for specialized facilities for carrying out the test.

The inventors of the present invention have devised a technique which, besides meeting the above-mentioned requirements, also has the advantage of being extremely cost-effective.

### DISCLOSURE OF INVENTION

The subject-matter of the present invention is a liquid analysis mixture for the diagnosis of urinary tract infections, whose essential features are set forth in claim 1, and whose preferred and/or auxiliary features are set forth in claims 2 and 3.

A further subject-matter of the present invention is a device useful for a colorimetric diagnosis of urinary tract infections, whose essential features are set forth in claim 4.

### BEST MODE FOR CARRYING OUT THE INVENTION

Below are some illustrative and non-limiting examples.

The device according to the present invention is made up of at least one vial comprising a liquid analysis mixture composed of a water part and an organic part immiscible with the water part and having a lower density than the same. The water part comprises a reagent mixture in turn comprising nutrient substances for possible bacterial culture, a redox indicator with a potential ranging from -100 to +500 mV and a buffer system able to maintain the pH in a range from 6.0 to 8.0.

In particular, the reagent mixture comprises from 94.0 to 99.9% by weight of nutrient substances and from 0.1 to 6.0% by weight of redox indicator.

The above indicates that the present invention refers to reagent mixtures comprising substances in specific weight ratios which, however, could also comprise other substances without departing from the scope of the present invention.

The nutrient substances comprise an amino acid source selected from the group consisting of meat infusions or peptones, vegetable peptones, casein hydrolysates, tryptose, tryptones and yeast extract, preferably in combination with a carbohydrate source selected among oligomeric or monomeric carbohydrates or metabolites thereof that can be metabolised by micro-organisms, such as glucose and lactose or intermediate metabolites thereof.

The redox indicator has the job of measuring the metabolism of the bacteria. In the presence of bacteria, and in a time proportional to the bacterial concentration, the redox indicator will go from the oxidised state to the reduced state, therefore changing the colour of the solution in which said indicator is present according to the number of vital bacteria present. Therefore, urine with a high vital bacteria content will cause a rapid change in the redox state of the indicator and, therefore, the colour of the water solution containing the reagents will change rapidly. Urine with a low vital bacteria content or no total bacteria, on the other hand, will not cause the redox state of the indicator to change or if it does, only over a very long period, and therefore during the observation times no change in colour of the water solution containing the reagents will be seen.

The redox indicator can be methylene blue or another redox indicator with a potential ranging from -100 to +500 mv, such as: dichlorophenol-indophenol; o-cresol-indophenol; thionine ("Lauth's violet"); methylene blue; indigotetrasulfonic acid; indigotrisulfonic acid; indigo carmine (indigodisulfonic acid); indigomonosulfonic acid; phenosafranin; safranin T; neutral red 5; tetramethylphenylenediamine; or combinations thereof.

Implementation of the invention requires a buffer system. In fact, the majority of redox indicators used in the present invention, in order to highlight the bacterial metabolism, have a redox potential dependent on the pH. A variation in the pH of the reaction medium would alter the change in the redox state of the indicator and, therefore, would falsify the colour change, preventing accurate detection of the number of bacteria present in the urine.

The presence of an organic phase is also necessary for implementation of the invention. In fact, the organic phase, by spontaneously stratifying above the water solution containing the urine to be tested and containing any bacteria together with the reagents described above, prevents diffusion of the atmospheric oxygen inside said water solution. In fact any atmospheric oxygen reaching the water phase would interact with the redox indicators, reoxidizing any that may have been reduced by the bacterial metabolism and, consequently, there would no longer be a colour change proportional to the number of vital bacteria present in the urine sample to be tested.

An example of an organic part is Vaseline oil.

The vial according to the present invention can comprise a cap having a tank delimited by a breakable wall and housing a disinfectant substance. In this way, once the test has ended, the vial can be disinfected by breaking the wall defining the tank. The aforesaid cap with tank is known and, therefore, it will not be described in detail.

Table I shows the compositions of sixteen reagent mixtures (1 - 16) in 10 ml of distilled water.

**TABLE I**

| | Indicator (g/l) | Aminoacid source (g/l) | Carbohydrate source(g/l) | Buffer (g/l) |
|---|---|---|---|---|
| 1 | Resazurin (0,2) | Tryptone (10) | Lactose (10) | Hepes (10) + TRIS (2.5) |
| 2 | Resazurin (0,2) | Meat infusion (10) | Lactose (10) | Hepes (10) + TRIS (2.5) |
| 3 | Resazurin (0,2) | Tryptone (10) | Glucose (10) | Hepes (10) + TRIS (2.5) |
| 4 | Resazurin (0,2) | Tryptone (10) | Lactose (10) | Mono-dibasic phosphate (15) |
| 5 | Dichlorophenol-indophenol (0.2) | Tryptone (10) | Lactose (10) | Hepes (10) + TRIS (2.5) |
| 6 | Dichlorophenol-indophenol (0.2) | Meat infusion (10) | Lactose (10) | Hepes (10) + TRIS (2.5) |
| 7 | Dichlorophenol-indophenol (0.2) | Tryptone (10) | Glucose (10) | Hepes (10) + TRIS (2.5) |
| 8 | Dichlorophenol-indophenol (0.2) | Tryptone (10) | Lactose (10) | Mono-dibasic phosphate (15) |
| 9 | Thionine (0.2) | Tryptone (10) | Lactose (10) | Hepes (10) + TRIS (2.5) |
| 10 | Thionine (0.2) | Meat infusion (10) | Lactose (10) | Hepes (10) + TRIS (2.5) |
| 11 | Thionine (0.2) | Tryptone (10) | Glucose (10) | Hepes (10) + TRIS (2.5) |
| 12 | Thionine (0,2) | Tryptone (10) | Lactose (10) | Mono-dibasic phosphate (15) |
| 13 | Tetramethylphenylenediamine (0.2) | Tryptone (10) | Lactose (10) | Hepes (10) + TRIS (2.5) |
| 14 | Tetramethylphenylenediamine (0.2) | Meat infusion (10) | Lactose (10) | Hepes (10) + TRIS (2.5) |
| 15 | Tetramethylphenylenediamine (0.2) | Tryptone (10) | Glucose (10) | Hepes (10) + TRIS (2.5) |
| 16 | Tetramethylphenylenediamine (0.2) | Tryptone (10) | Lactose (10) | Mono-dibasic phosphate (15) |

The reagent mixtures were prepared by checking that the pH was 7.0, thus producing the water part of the liquid analysis mixture subject of the present invention.

Each one of the reagent mixtures of Table I was used to prepare a respective liquid analysis mixture by the addition of 1.5 ml of Vaseline oil as the organic part.

Each of the above vials was tested with samples of urines that had been artificially contaminated with two different concentrations (10² and 10⁷ CFU/ml) of pathogenic bacteria, such as: *Escherichia coli* (ATCC 25992), *Enterococcus faecalis* (ATCC 29212), *Pseudomonas aeruginosa* (ATCC 27853), *Staphylococcus aureus* (ATCC 12600). Said pathogenic bacteria are the ones that most frequently cause urinary tract infections. In particular, the micro-organisms were added in concentrations that are equal to the ones that are normally detected in urinary tract infections (>10⁶ CFU/ml) or in concentrations that are smaller than the ones that are normally detected in urinary tract infections (<10³ CFU/ml) . For each vial, the test involved the addition of 0.5 ml of urine artificially contaminated as described above. The vial thus prepared was incubated at a temperature of 37°C (+ 0.5°C) and observed at regular time intervals, recording the time necessary for the colour change.

The colorimetric diagnosis tests with the devices subject of the present invention can be carried out using a urine sample varying from 0.5 µl to 10 ml.

Tables II and III show the values in hours of each of the tests carried out.

**Table II**

| | *Enterococcus faecalis* | | *Escherichia coli* | |
|---|---|---|---|---|
| | 10⁷ CFU/ml | 10² CFU/ml | 10⁷ CFU/ml | 10² CFU/ml |
| 1 | 6.00 | >18 | 4.33 | >18 |
| 2 | 3.35 | >18 | 6.00 | >18 |
| 3 | 6.00 | >18 | 4.70 | >18 |
| 4 | 4.20 | >18 | 4.33 | >18 |
| 5 | 3.33 | >18 | 6.00 | >18 |
| 6 | 4.60 | >18 | 4.70 | >18 |
| 7 | 6.00 | >18 | 4.40 | >18 |
| 8 | 3.90 | >18 | 6.00 | >18 |
| 9 | 6.00 | >18 | 4.80 | >18 |
| 10 | 4.40 | >18 | 4.40 | >18 |
| 11 | 4.40 | >18 | 4.40 | >18 |
| 12 | 11.83 | >18 | 5.08 | >18 |
| 13 | 9.30 | >18 | 6.00 | >18 |
| 14 | 9.30 | >18 | 5.00 | >18 |
| 15 | 9.40 | >18 | 5.30 | >18 |
| 16 | 8.00 | >18 | 6.00 | >18 |

**Table III**

| | *Pseudomonas aeruginosa* | | *Staphylococcus aureus* | |
|---|---|---|---|---|
| | 10⁷ CFU/ml | 10² CFU/ml | 10⁷ CFU/ml | 10² CFU/ml |
| 1 | 7.30 | >18 | 10.20 | >18 |
| 2 | 10.50 | >18 | >18 | >18 |
| 3 | 7.00 | >18 | 8.55 | >18 |
| 4 | 7.15 | >18 | 9.10 | >18 |
| 5 | 8.30 | >18 | 10.45 | >18 |
| 6 | 6.5 | >18 | 8.60 | >18 |
| 7 | 10.00 | >18 | 8.20 | >18 |
| 8 | >18 | >18 | >18 | >18 |
| 9 | 6.80 | >18 | 9.25 | >18 |
| 10 | 7.20 | >18 | 10.45 | >18 |
| 11 | 8.00 | >18 | >18 | >18 |
| 12 | 6.38 | >18 | 12.07 | >18 |
| 13 | 6.20 | >18 | 11.60 | >18 |
| 14 | 6.00 | >18 | 10.85 | >18 |
| 15 | 6.80 | >18 | 10.50 | >18 |
| 16 | 8.30 | >18 | 12.00 | >18 |

The values shown in Tables II and III prove that the technique according to the present invention ensures a quick and effective response to the bacterial load present in urine samples.

The variability in terms of time necessary to observe the colour change can be partly attributed to the differences in the composition of the relative reagent mixture and partly to the differences in culture and metabolism of the different bacteria used.

It should be underlined that for implementation of the present invention, it must be possible for the colour change due to the change in the redox state of the redox indicator, in turn caused by the bacterial metabolism, to be observed only in liquid, i.e. in a water solution contained inside a vial with transparent walls, hence the invention cannot be used on ordinary solid agar medium for microbiology.

The colorimetric response could be supported by the use of a reader device - known and, therefore, not described in detail - which is able to read the colour change of the vial and to translate it into a signal and/or a value.

Some of the vials of the device according to the present invention further comprise an antibiotic for the purpose of testing the effectiveness thereof towards the possible bacterial load present in the urine sample being tested. If the vial without antibiotic detects the presence of a bacterial load, the other vials comprising the different antibiotics highlight which one of them is effective. In fact, after having established the presence of the bacterial load, the vial with antibiotic that does not show any colour change is the one that necessarily contains the effective antibiotic.

## Claims

1. A liquid analysis mixture for a colorimetric diagnosis of urinary tract infections said liquid analysis mixture comprising a water part and an organic part, said water part comprising a reagent mixture comprising
(a) nutrient substances comprising an amino acid source selected from the group consisting of meat infusions or peptones, vegetable peptones, casein hydrolysates, tryptose, tryptones and yeast extract;
(b) at least one redox indicator with a potential ranging from -100 to +500 mV; and
(c) a buffer system able to maintain the pH in a range from 6.0 to 8.0;
said reagent mixture comprising from 94.0 to 99.9% by weight of the nutrient substances and from 0.1 to 6.0% by weight of the redox indicator,
**characterized in that** the organic part is immiscible with the water part and has a density lower than that of the water part.

2. A liquid analysis mixture according to claim 1, wherein said nutrient substances comprise a carbohydrate source chosen from oligomeric or monomeric carbohydrates or metabolites thereof, that can be metabolised by micro-organisms.

3. A liquid analysis mixture according to one of the claims 1 or 2, wherein said reagent mixture comprises an antibiotic.

4. A device for a colorimetric diagnosis of urinary tract infections, said device comprising at least one vial wherein the liquid analysis mixture of any of claims 1 to 3 is housed.

## Patentansprüche

1. Flüssiges Analysegemisch für eine kolorimetrische Diagnose von Infektionen des Harntrakts, wobei das flüssige Analysegemisch einen Wasserteil und einen organischen Teil umfasst, wobei der Wasserteil ein Reagensgemisch umfasst, welches umfasst
(a) Nährstoffstubstanzen, umfassend eine Aminosäurequelle, ausgewählt aus der Gruppe, bestehend aus Fleischinfusum (meat infusions) oder -peptonen, pflanzlichen Peptonen, Kaseinhydrolysaten, Tryptose, Tryptonen und Hefeextrakt;
(b) wenigstens einen Redox-Indikator mit einem Potential im Bereich von -100 bis +500 mV; und
(c) ein Puffersystem, welches den pH-Wert in einem Bereich von 6,0 bis 8,0 aufrechterhalten kann;
wobei das Reagensgemisch von 94,0 bis 99,9 Gewichts-% der Nährstoffsubstanzen und von 0,1 bis 6,0 Gewichts-% des Redox-Indikators umfasst,
**dadurch gekennzeichnet, dass** der organische Teil mit dem Wasserteil nicht mischbar ist und eine Dichte hat, die geringer ist als die des Wasserteils.

2. Flüssiges Analysegemisch gemäß Anspruch 1, wobei die Nährstoffsubstanzen eine Kohlenhydratquelle umfassen, ausgewählt aus oligomeren oder monomeren Kohlenhydraten oder Metaboliten davon, die durch Mikroorganismen metabolisiert werden können.

3. Flüssiges Analysegemisch gemäß einem der Ansprüche 1 oder 2, wobei das Reagensgemisch ein Antibiotikum umfasst.

4. Vorrichtung für eine kolorimetrische Diagnose von Infektionen des Harntrakts, wobei die Vorrichtung wenigstens ein Fläschchen umfasst, worin das flüssige Analysegemisch nach irgendeinem der Ansprüche 1 bis 3 aufgenommen ist.

## Revendications

1. Mélange d'analyse liquide pour un diagnostic colorimétrique d'infections des voies urinaires, ledit mélange d'analyse liquide comprenant une partie aqueuse et une partie organique, ladite partie aqueuse comprenant un mélange de réactifs comprenant
(a) des substances nutritives comprenant une source d'acides aminés choisie dans le groupe constitué par les infusions ou peptones de viande, les peptones végétales, les hydrolysats de caséine, le tryptose, les tryptones et l'extrait de levure ;
(b) au moins un indicateur redox ayant un potentiel situé dans la plage allant de -100 à +500 mV ; et
(c) un système tampon capable de maintenir le pH dans une plage allant de 6,0 à 8,0 ;
ledit mélange de réactifs comprenant de 94,0 à 99,9 % en poids des substances nutritives et de 0,1 à 6,0 % en poids de l'indicateur redox,
**caractérisé en ce que** la partie organique n'est pas miscible avec la partie aqueuse et a une densité inférieure à celle de la partie aqueuse.

2. Mélange d'analyse liquide selon la revendication 1, dans lequel lesdites substances nutritives comprennent une source d'hydrates de carbone choisie parmi les hydrates de carbone oligomères et monomères ainsi que leurs métabolites, qui peuvent être métabolisés par des micro-organismes.

3. Mélange d'analyse liquide selon l'une des revendications 1 et 2, dans lequel ledit mélange de réactifs comprend un antibiotique.

4. Dispositif pour le diagnostic colorimétrique d'infections des voies urinaires, ledit dispositif comprenant au moins un flacon dans lequel se trouve le mélange d'analyse liquide de l'une quelconque des revendications 1 à 3.
